# EUROPEAN PATENT APPLICATION

(11) **EP 1 900 370 A1**
(43) Date of publication of application: **19.03.2008**
(21) Application number: 07253529.7
(22) Date of filing: 06.09.2007
(51) Int. Cl.: A61K 35/28, A61K 38/36, A61K 38/18, A61K 38/22, A61L 24/10

(54) **Composition for repairing nerve damage**

(30) Priority: 06.09.2006 US 516378
(71) Applicant: Cheng, Henrich, Pei-Tou District Taipei (TW)
(72) Inventor: Cheng, Henrich, Taipei (TW); Huang, Shiang-Suo, Taipei (TW); Tsai, Shen-Kou, Taipei (TW)
(74) Representative: Wright, Simon Mark

(57) **Abstract**

The present invention relates to a fibrin glue or topically applicable composition, for repairing nerve damage and/or enhancing the functional recovery of a damaged nerve, which includes fibrin glue and an amount of bone marrow stem cells (BMSCs) effective for repairing the nerve damage and/or enhancing at least partially the functional recovery. The composition can be used in a method for repairing nerve damage and/or enhancing the functional recovery of a damaged nerve in a subject by topically applying it to the damaged nerve.

## Description

### FIELD OF THE INVENTION

The present invention relates to a composition for repairing nerve damage and enhancing functional recovery of a damaged nerve, as well as methods utilizing the same.

### BACKGROUND OF THE INVENTION

Nerve damage is usually caused by trauma or ischemia, and is difficult to repair. A vertebrate having nerve damage may suffer from motor deficits, paralysis, or even death. Ischemia is caused by abrupt interruption of cerebral blood flow that leads to cerebral infarction and ischemic changes with long-term motor deficits (S.I. Savitz et al., 2002, Ann. Neural. 52: 266-275). Loss of neural cells after ischemic injury to the central nervous system (CNS) makes CNS repair difficult.

Many studies showed that neural stem cells (NSCs) isolated from various rodent and human CNS areas are capable of differentiating into neural cells in the adult rodent CNS under the influence of the environment and/or exogenous growth factors (F.H. Gage, 2000, Science 287: 1433-1438; J. Price and B.P. Williams, 2001, Curr. Opin. Neurobiol. 11: 564-567). Thus, replenishment of NSCs is thought to be a potential strategy for human CNS treatment.

Stem cells derived from human bone marrow are heterogeneous in morphology. They multipotentially differentiate into osteoblasts, adipocytes, chondrocytes and muscle and can also generate neurons (E. Mezey et al., 2000, Science 290: 1779-1782; E. Mezey et al., 2003, Proc. Natl. Acad. Sci. USA. 100: 1364-1369; J.R. Sanchez-Ramos et al., 2000, Exp. Neural. 164: 247-256; D. Woodbury et al., 2000, J. Neurosci. Res. 61: 364-370). Recently, human and mouse bone marrow stem cells (BMSCs) have been reported to express neuronal progenitor marker (nestin), neuron-specific nuclear protein (NeuN), and glial acidic fibrillary protein (GFAP) after stimulation with retinoic acid and epidermal growth factor (EGF) or brain-derived neurotrophic factor (BDNF) (J.R. Sanchez-Ramos *et al.,* 2000, *supra*). It has also been demonstrated that transplanted BMSCs are able to differentiate between the neuronal and glial lineages in damaged CNS (J.R. Sanchez-Ramos, 2002, J. Neurosci Res. 69: 880-893). Moreover, it has been found that the transplantation of BMSCs can improve functional recovery in rats with focal cerebral ischemia (J. Chen et al., 2000, Neuropharmacology 39: 711-716), in rats with traumatic brain injury (D. Lu et al., 2001, J. Neurotrauma 18: 813-819), and in mice with Parkinson's disease (Y. Li et al., 2001, Neurosci Lett. 316: 67-70).

In the aforementioned studies, the routes of administration of BMSCs are via intravenous or intracerebral injection. There has been no literature reporting the effect of topical application of BMSCs on damaged CNS areas in test animals subjected to nerve damage caused by, for example, chronic focal cerebral ischemic injury. Moreover, there remains a need for a means to effectively repair nerve damage and as a result thereof, enhance the functional recovery of damaged nerves.

### BRIEF SUMMARY OF THE INVENTION

One aim of the present invention is to provide compositions and methods for effectively repairing nerve damage and, as a result thereof, enhancing at least partially the functional recovery of the damaged nerve. The composition may be adapted for topical application and may solidify, or be solidifiable, in use. It is preferably a fibrin glue composition.

Accordingly, in a first aspect, the present invention relates to a (e.g. fibrin glue) topically applicable composition for repairing nerve damage and/or enhancing functional recovery of a damaged nerve. This may comprise fibrin glue and/or (an amount of) bone marrow stem cells (BMSCs), suitably effective to repair the nerve damage. In a preferred embodiment of the present invention, the (fibrin glue) composition may further comprise (an amount of) a nerve growth factor effective to repair the nerve damage.

Also, in the first aspect, the present invention relates to a (fibrin glue or topically applicable) composition for enhancing functional recovery of a damaged nerve. This may comprise fibrin glue and/or (an amount of) BMSCs, suitably effective to enhance at least partially the functional recovery of the damaged nerve. In a preferred embodiment of the present invention, the (fibrin glue) composition may further comprise (an amount of) a nerve growth factor effective to enhance at least partially the functional recovery of the damaged nerve.

In a further aspect, the present invention uses the composition of the first aspect in a method for repairing nerve damage in a subject. The method can comprise providing a fibrin glue or topically applicable composition comprising fibrin glue and (an amount of BMSCs), suitably effective to repair the nerve damage, and topically applying the composition to the damaged nerve. In a preferred embodiment of the present invention, the (fibrin glue) composition may further comprise (an amount of) a nerve growth factor effective to repair the nerve damage.

Similarly the present invention uses the compositions of the first aspect in a method for enhancing functional recovery of a damaged nerve in a subject. The method comprises providing a fibrin glue or topically applicable composition comprising fibrin glue and (an amount of) BMSCs, suitably effective to enhance at least partially the functional recovery of the damaged nerve, and topically applying the composition to the damaged nerve. In a preferred embodiment of the present invention, the (fibrin glue) composition may further comprise (an amount of) a nerve growth factor effective to enhance at least partially the functional recovery of the damaged nerve.
The invention thus relates to the use of a composition of the first aspect, optionally in the manufacture of a medicament, for repairing nerve damage and/or enhancing functional recovery of a damaged nerve.

### BRIEF DESCRIPTION OF THE SEVERAL VIEWS OF THE DRAWINGS

The foregoing summary, as well as the following detailed description of the invention, will be better understood when read in conjunction with the appended drawings. For the purpose of illustrating the invention, there are shown in the drawings embodiments which are presently preferred. It should be understood, however, that the invention is not limited to the precise arrangements and instrumentalities shown.

In the drawings:

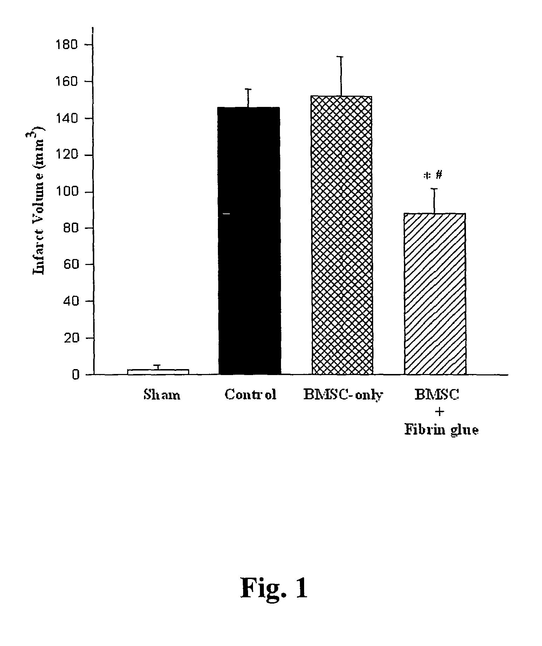

Fig. 1 shows the infarct volumes assessed after one hour of middle cerebral artery (MCA) occlusion and five weeks of reperfusion by vital 2,3,5-triphenyltetrazolium chloride (TTC) dye staining. The infarct volume of the size-sieved stem cells (SSCs)-fibrin glue group was significantly reduced as compared with the control group and the SSCs-only group. Each experimental group consisted of 6 rats. Results are expressed as mean ± SEM. *, *p*<0.05 when compared with the control group. ^{#}, *p*<0.05 when compared with the SSCs-only group.

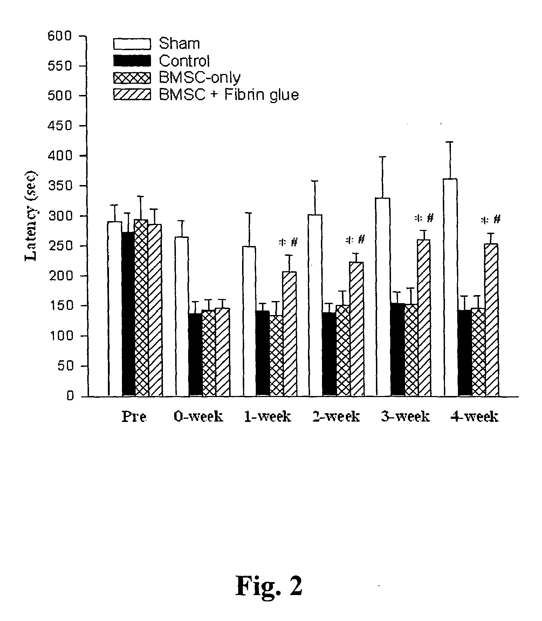

Fig. 2 shows the effect of SSCs in the rotarod test on rats after focal cerebral ischemic (FCI) injury. The results are expressed as durations (in second) spent on the rotarod test. Sham-operated animals without ischemia stayed significantly longer on the rotarod than other groups. "0-week" indicates the time of topically applying the SSCs. In the SSCs-fibrin glue group, the duration of rats stayed on the rotarod was significantly increased as compared with the control group and the SSCs-only group at the end of the 1st, 2nd, 3rd and 4th week after SSCs administration. Each experimental group consisted of 6 rats. Results are expressed as mean ± SEM. *, p<0.05 when compared with the control group. ^{#}, *p*<0.05 when compared with the SSCs-only group.

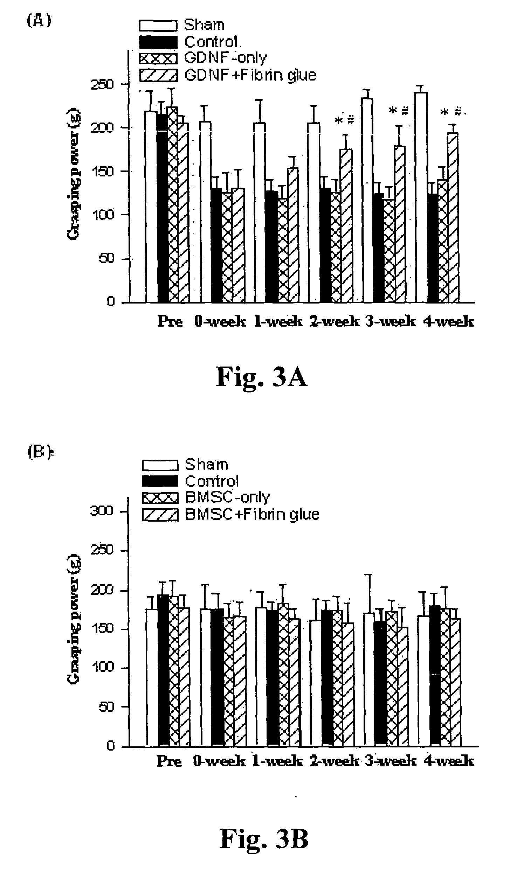

Fig. 3 comprises Figs. 3A and 3B, and shows the effect of SSCs on the grasping power of (Fig. 3A) right forepaw and (Fig. 3B) left forepaw of rats after FCI injury. "0-week" indicates the time of topically applying the SSCs. There was no significant difference on the grasping power of the right forepaw among the four groups. In the SSCs-fibrin glue group, the grasping power of the left forepaw of rats was significantly increased as compared with the control group and the SSCs-only group at the end of the 2nd, 3rd and 4th week after SSCs administration. Each experimental group consisted of 6 rats. Results are expressed as mean ± SEM. *, *p*<0.05 when compared with the control group. *^{#}, p<0.05* when compared with the SSCs-only group.

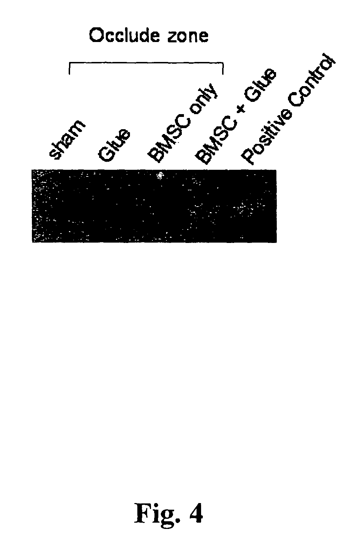

Fig. 4 shows the representative PCR analysis results for human glycerol-3-phosphate dehydrogenase (HG3PDH) of an animal topically applied SSCs (10⁶ cells) in fibrin glue (i.e., SSCs-fibrin glue) one week after FCI injury and five weeks of reperfusion. Postive control is 60 pg of human DNA extracted from SSCs. The pattern shown is representative of three independent experiments.

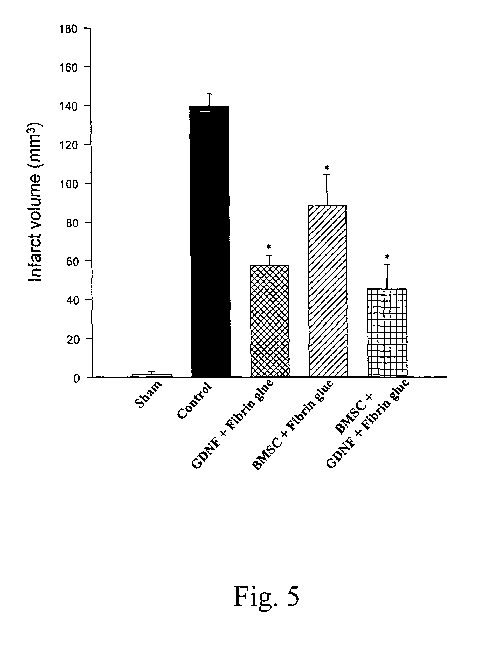

Fig. 5 shows the infarct volumes assessed after one hour of MCA occlusion and five weeks of reperfusion by vital TTC staining. The infarct volumes of the glial cell line-derived neurotrophic factor (GDNF)-fibrin glue group, the SSCs-fibrin glue group, and the GDNF-SSCs-fibrin glue group were significantly reduced as compared with the control group. Each experimental group consisted of 6 rats. Results are expressed as mean ± SEM. *, *p*<0.05 when compared with the control group.

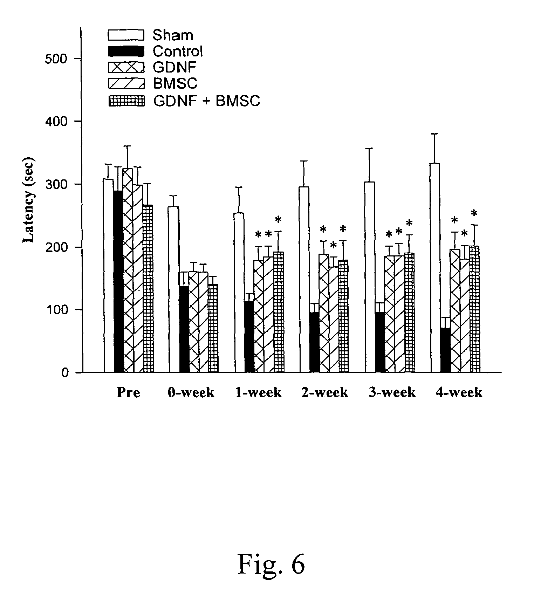

Fig. 6 shows the effect of SSCs + GDNF in the rotarod test on rats after FCI injury. The results are expressed as durations (in seconds) spent on the rotarod test. The duration that the animals stayed on the rotarod was not significantly different among the five groups before surgical preparation. Sham-operated animals without ischemia stayed significantly longer on the rotarod test than other groups after FCI injury. "0-week" indicates the time of topically applying the GDNF, SSCs or GDNF + SSCs. In the GDNF-fibrin glue group, the SSCs-fibrin glue group, and the GDNF-SSCs-fibrin glue group, the duration that the rats stayed on the rotarod was significantly increased as compared with the control group at the end of the 1 st, 2nd, 3rd and 4th week after GDNF, SSCs or GDNF + SSCs administration. Each experimental group consisted of 6 rats. Results are expressed as mean ± SEM. *, *p*<0.05 when compared with the control group.

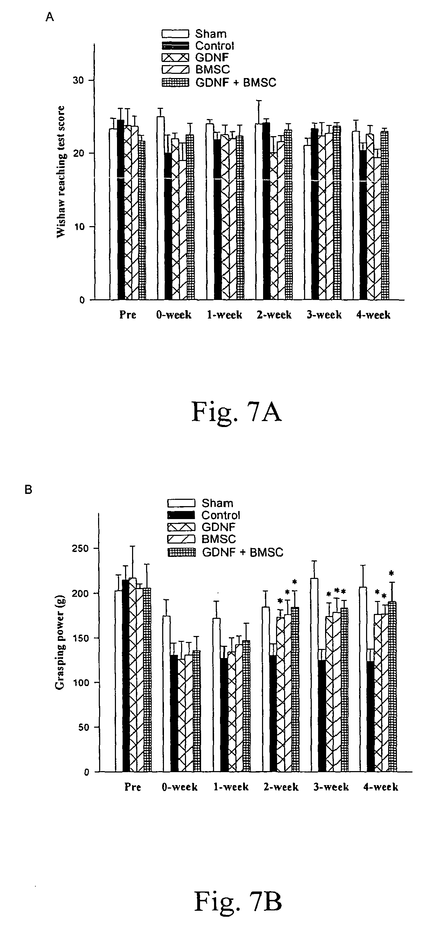

Fig. 7 comprises Figs. 7A and 7B and shows the effect of SSCs + GDNF on the grasping power of (Fig. 7A) right forepaw and (Fig. 7B) left forepaw of rats after FCI injury. "0-week" indicates the time of topically applying the GDNF, SSCs or GDNF + SSCs. Among the five groups, there is no significant difference in the mean grasping power of the left forepaws of rats before the right MCA occlusion, or in the right forepaws of rats before or after right MCA occlusion. In the GDNF-fibrin glue group, the SSCs-fibrin glue group, and the GDNF-SSCs-fibrin glue group, the grasping power of left forepaw of rats was significantly increased as compared with the control group at the end of the 2nd, 3rd and 4th week after GDNF, SSCs or GDNF + SSCs administration. Each experimental group consisted of 6 rats. Results are expressed as mean ± SEM. *, *p<0.05* when compared with the control group.

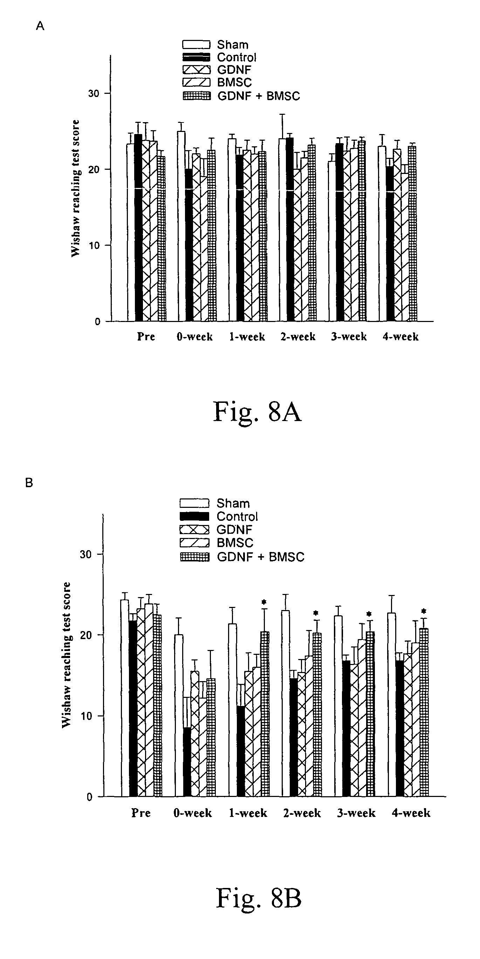

Fig. 8 comprises Figs. 8A and 8B and shows the effect of SSCs + GDNF in the whishaw reaching test of (A) right forepaw and (B) left forepaw of rats after FCI injury. "0-week" indicates the time of topically applying the GDNF, SSCs or GDNF + SSCs. In the GDNF-fibrin glue group and the SSCs-fibrin glue group, there is no significant difference in the test rats before or after the right MCA occlusion. In the GDNF-SSCs-fibrin glue group, the scores of rats on the test were significantly increased as compared with the control group at the end of the 1st, 2nd, 3rd and 4th week after GDNF + SSCs administration. Each experimental group consisted of 6 rats. Results are expressed as mean ± SEM. *, *p*<0.05 when compared with the control group.

### DETAILED DESCRIPTION OF THE INVENTION

The present invention relates to a topical, e.g. fibrin glue, composition for repairing nerve damage and/or enhancing the functional recovery of a damaged nerve which comprises fibrin glue and/or (an amount of) bone marrow stem cells (BMSCs), suitably effective to repair the nerve damage and/or enhancing at least partially the functional recovery of the damaged nerve.

According to the present invention, BMSCs are stem cells derived from human bone marrow and can have the potential of differentiating into neurons. They are thus considered to be good material for regenerating neural cells. Preferably, the BMSCs used in the present invention are size-sieved stem cells (SSCs) preferably derived from human bone marrow.

SSCs can be developed based on their different sizes and specific surface markers, in order to generate homogeneous populations, suitably by using cell sorting (to avoid heterogeneous population generation of primary BMSC cultures). SSCs have greater renewal capability than a heterogeneous population of BMSCs. In a preferred embodiment of the present invention, the SSCs are isolated as a homogeneous population from human bone marrow on the basis of cell size and adherent capacity via Percoll gradient separation (and a 3-µm porous sieve for discarding smaller cells as described by S.C. Hung et al., 2002, Stem Cells 20: 249-258).

According to the present invention, SSCs may be induced for neural differentiation before addition to or incorporation into the fibrin glue composition. The induction may be achieved by treating the SSCs with antioxidant agents, such as β-mercaptoethanol and retinoic acid, which are often used *in vitro* to induce the neural differentiation of stem cells (S.C. Hung et al., 2002, Stem Cells 20: 522-529). In a preferred embodiment of the present invention, the SSCs are induced by a neurotrophic factor, such as glial cell line-derived neurotrophic factor (GDNF) or pituitary adenylate cyclase-activating polypeptide (PACAP), and/or dibutyryl cAMP (dbcAMP) as described in the copending US patent application Ser. No. 10/873,640, filed June 23, 2004 (Pub. No. 2005 0287665).

As used herein, the term "effective amount" refers to an amount of the BMSCs, and, when optionally used, the nerve growth factor, in the fibrin glue composition of the present invention, which, when applied to a subject suffering from nerve damage, attains a desired effect, i.e., it can repair nerve damage in the subject and/or enhance at least partially functional recovery of a damaged nerve. The effective amount can be readily determined in view of the present disclosure by persons of ordinary skill in the art without undue experimentation. Preferably, an effective amount of BMSCs (applied to a target area in the present invention) is from 10⁵ to 10⁷ cells. More preferably, the effective amount is from 5 x 10⁵ to 5 x 10⁶, such as about 10⁶ cells. Preferred and more preferred concentrations of the nerve growth factor, when it is used with the BMSCs, are set forth below.

The term "fibrin glue" as used herein can be a biocompatible and biodegradable product. It may suitably comprise, or be formed by, fibrinogen (and one or more other reagents). Fibrin glue may comprise fibrinogen, and thrombin and/or aprotinin; it has been used customarily as an adhesive agent in various kinds of surgery, including neurosurgery. On contact, the thrombin converts the fibrinogen to fibrin. It is commercially available under the trade mark Beriplast P™ (ZLB or Aventis Behring, Germany), for example. It is suitably capable of forming a fibrin clot.

The fibin glue used in the present invention preferably comprises, or is composed, of three (main) ingredients: fibrinogen, aprotinin and a calcium source able to provide divalent calcium ions (such as calcium chloride or calcium carbonate). To apply the fibrin glue, fibrinogen can be first mixed with aprotinin. It can then be further mixed with the calcium source (in the surgical, affected or relevant area), usually to form a glue cast.

To make (e.g. fibrin glue composition) comprising BMSCs, the BMSCs are preferably mixed with the fibrinogen and aprotinin before mixing with the calcium source, e.g. chloride (in the surgical area).

In a fibrin glue composition of the present invention, the concentration of fibrinogen may be in the range of from 10 mg/ml to 1000 mg/ml, preferably from 50-200 or 80-120 mg/ml and more preferably about 100 mg/ml. The concentration of aprotinin may be in the range of about 10 KIU/ml to about 500 KIU/ml, preferably from 100-300 or 150-250 KIU/ml, more preferably about 200 KIU/ml. The concentration (e.g. of calcium chloride) used, or of Ca²⁺ as the calcium source, may be in the range of from 1 mM to 100 mM, preferably 4-15 or 6-10 mM, more preferably about 8 mM.

The fibrin glue composition of the present invention may further comprise, in addition to the fibrin glue components (referred to herein as the "fibrin glue") and BMSCs, (an effective amount of) a nerve or fibroblast growth factor. The nerve growth factor used in the composition of the present invention may be selected from, but not limited to, glial cell line-derived neurotrophic factor (GDNF), transforming growth factor-beta, fibroblast growth factor, platelet-derived growth factor, epidermal growth factor, vascular endothelial growth factor (VEGF), and neurotrophin (such as nerve growth factor (NGF), brain-derived neurotrophic factor (BDNF), NT3, NT4 and NT5). Most preferably, the nerve growth factor is glial cell line-derived neurotrophic factor (GDNF).

According to the invention, the concentration of the nerve growth factor in the fibrin glue composition of the invention may preferably be about 1 µg/ml to 1000 µg/ml, and more preferably from 30-100, such as about 50 µg/ml in the composition. The concentration of (e.g. acidic) fibroblast growth factor may be from 0.001 to 1000 mg/ml, such as from 0.1 or 0.5 to 10 or 20 mg/ml, for example about 1 mg/ml.

The fibrin glue composition of the invention is suitable for use in repairing all kinds of nerve damages and enhancing the functional recovery of damaged nerves in all nerve systems, including the central nervous system, peripheral nervous system, sympathetic nervous system, and parasympathetic nervous system. Preferably, the damaged nerve is in the central nervous system. In an embodiment of the present invention, the nerve damage is caused by focal cerebral ischemia (FCI). In a preferred embodiment, the nerve damage is caused by chronic focal cerebral ischemia.

As used herein, the term "repairing nerve damage" and other grammatically equivalent terms, refer to an improvement in the pathological conditions of the subject suffering from nerve damage. The term "functional recovery of a damaged nerve" refers to restoration, at least partially, of the physical function of the damaged nerve. For example, for nerve damage caused by FCI, the reduction in total infarction volume and motor deficits are signs of the repair of the damaged nerves and restoration of the function thereof. In an animal model according to the present invention, the reduction in motor deficits comprises improvement in balance, coordination and grasping strength.

As used herein, the singular forms "a", "an", and "the" include plural references unless the context clearly dictates otherwise. Thus, for example, reference to a damaged nerve refers to one or more damaged nerves.

As used herein, the term "subject" includes a (e.g. non-human) mammal, including a pet or domestic animal, such as a cat or a dog, a laboratory animal, such as a mouse or a rat, and livestock, such as a horse, a cow, etc.; and preferably a human.

The fibrin glue composition of the present invention can be topically applied to damaged nerves. The topical application may be practiced, for example, during surgery, that exposes the nerve to topical administration by coating the area including the nerve with the glue from a syringe.

The method according to the present invention may provide a long-term effect needed for repairing chronic nerve damage and enhancing the functional recovery of the damaged nerve. In addition, topical application of the composition of the present invention to a damaged nerve only once may sufficiently achieve the desired effect of functional recovery. Preferably, the method further comprises preparing the fibrin glue composition (freshly), just before use.

The composition and method of the present invention may be used in the treatment of various nerve damage-related diseases, including, but not limited to, ischemic brain disease. Preferably, the ischemic brain disease comprises stroke, thrombosis and embolization of the middle cerebral artery (MCA).

**Examples**

The present invention is further illustrated by the following examples, which are provided for the purpose of demonstration rather than limitation.

*Animals*

The following experiments conform to the Guide for the Care and Use of Laboratory Animals published by the US National Institutes of Health (NIH publication NO. 85-23, revised 1996). Male Long-Evans rats (National Lab. Animal Breeding and Research Center) weighing 300g-350g were used in the following experiments. The animals were housed in a room with controlled temperature (24 ± 1 °C) and humidity (55 ± 5%) under a 12:12 h light-dark cycle. They were allowed free access to food and water.

*Size-sieved Stem Cells (SSCs)*

SSCs used in the following experiments were isolated from human bone marrow as previously reported (S.C. *Hung et al.,* 2002, *supra*). In brief, bone marrow aspirated from the iliac crest of normal donors was washed twice with phosphate-buffered saline (PBS) and loaded onto 1.073 g/ml Percoll solution (Sigma), and then centrifuged at 900 × g for 30 mins. The cells were suspended in Dulbecco's modified Eagle's medium-low glucose (DMEM-LG) supplemented with 10% fetal bovine serum (FBS), 100 U/ml penicillin, 100 mg/ml streptomycin, and 0.25 µg/ml amphotericin B, and were plated in a 10-cm plastic culture dish comprising a plate with 3-µm pores (Transwell System, Corning) at a density of 10⁶ mononuclear cells/cm². SSCs that adhered to the pore-containing plate were recovered on the 7th day after initial plating, plated at about 6,000 cells/cm², and subcultured at a ratio of 1:3 until cells reached more than 80% confluence.

Undifferentiated SSCs were cultured in DMEM-LG supplemented with 10% FBS.
When SSCs grown in the serum containing medium reached 80% confluence, the SSCs were seeded at a density of 4,000 cells/cm², treated in serum medium (ITS medium) containing GDNF (20 ng/ml, RBI, Natick, MA, USA) for 48 hours, and then treated with serum depletion for 5 hours to 3 days to induce differentiation. ITS medium consisted of 56% DMEM-LG (Life Biotech), 40% MCDB-201 medium (Sigma), and 1 x ITS medium supplement (Sigma) that contained 1 mg/ml insulin, 0.55 mg/ml human transferrin, 0.5 µg/ml sodium selenite, 10 nM dexamethasone (Sigma), and 10 µM ascorbic acid (Sigma).

*Statistics*

Data acquired in the following experiments are expressed as mean ± standard error of mean (S.E.M.). Statistical analysis of the differences in volume of infarcts and the behavioral deficit scores of rats between control and treatment groups were carried by one-way analysis of variance (ANOVA) followed by unpaired, two-tailed *t* tests for combined data. A value of *P* < 0.05 was considered to be statistically significant.

**Example 1 Generation of Cerebral Infarction and Infarct Volume Analysis**

The technique used was a modification of the method of Huang et al. (S.S. Huang et al., 2001, Free Radic. Biol. Med. 30: 643-649). In brief, each male Long-Evans rat was anesthetized by inhalation of a nitrous oxide/oxygen/halothane (69%:30%:1%) mixture during surgical preparation. Body temperature was maintained during surgery at 37 ± 0.5°C with a heating pad servo-controlled by a rectal probe. The ventral tail artery was cannulated for continuous monitoring of heart rate and mean arterial blood pressure (MABP) by a Statham P23 XL transducer and displayed on a Gould RS-3400 physiological Recorder (Gould, Cleveland, OH, USA) and the pH, PO₂ and PCO₂ in the blood were tested using blood sampling with Blood Gas Analyzer (GEM-5300 I.L. CO, USA). Measurements were performed before, during, and just after unilateral middle cerebral artery (MCA) occlusion.

Focal cerebral ischemic (FCI) infarcts were produced in the right lateral cerebral cortex in the territory of MCA. Both common carotid arteries were exposed by midline anterior cervical incision. The animal was placed in a lateral position, and a skin incision was made at the midpoint between the right lateral canthus and the anterior pinna. The temporal muscle was retracted, and a small (3-mm diameter) craniectomy was made at the junction of the zygoma and squamosal bone using a drill (Dremel Multipro+5395, Dremel com. USA) cooled with saline solution. Using a dissecting microscope (OPMI-1, ZISS, Germany), the dura was opened with fine forceps, and the right MCA was ligated with 10-0 monofilament nylon ties. Both common carotid arteries were then occluded by microaneurysm clips for 1 hr. After removing the clips, return of flow was visualized in the arteries.

One week following the FCI injury, rats were assigned, in randomized sequence, to one of four treatment groups (n=6 each): (a) the sham group: animals underwent the same described surgical procedures except MCA ligation; (b) the control group: topically applied fibrin glue; (c) the SSCs-only group: topically applied SSCs (10⁶ cells); and (d) the SSCs (10⁶ cells)-fibrin glue group: topically applied fibrin glue containing SSCs (10⁶ cells). Topical application, in all instances was by coating the area including the nerve with the glue, with or without SSCs, from a syringe.

The fibrin glue (Beriplast P™, ZLB Behring, Germany) used in this experiment and customarily used as an adhesive agent in CNS tissue, was prepared before use by mixing the fibrinogen (100 mg/ml) with an aprotinin solution (200 KIU/ml). This solution was then mixed with calcium chloride (8 mM) in the surgical area to form a glue cast. The final volume of the solution locally applied in the infarcted brain tissue was 20 µl.

After focal cerebral ischemia for 1 hr and reperfusion for 5 weeks, the rats were anesthetized and killed by rapid decapitation. Brains were removed, inspected visually for the anatomy of the MCA and for signs of hemorrhage or infection, immersed in cold saline solution for 10 minutes, and sectioned into standard coronal slices (each 2-mm thick) using a brain matrix slicer (JACOBOWITZ Systems, Zivic-Miller Laboratories INC, Allison park, USA). Slices were placed in the vital dye 2,3,5-triphenyltetrazolium chloride (TTC, 2%; Sigma, USA) at 37°C in the dark for 30 minutes, followed by 10% formalin at room temperature overnight. The outline of right and left cerebral hemispheres as well as that of infarct tissue, clearly visualizable by TTC staining (S.T. Chen et al., 1986, Stroke 17: 738-743), was outlined on the posterior surface of each slice using an image analyzer (color image scanner, EPSON), connected to an image analysis system (AIS software, Imaging research INC, Canada), which was run on a personal computer (AMD K6-2 3D 400). The area of infarction was measured by subtracting the area of the non-lesioned ipsilateral hemisphere from that of the contralateral side. Infarct volume was calculated as the sum of infarct area per slice multiplied by slice thickness. Both the surgeon and image analyzer operator were blinded to the treatment given each animal.

Reproducible brain infarcts were obtained from a territory of right MCA occlusion in the SSCs-fibrin glue group, the SSCs-only group, and the control group. The infarct volume at the end of the 5th week after FCI injury in the SSCs-fibrin glue group was significantly reduced (88.1±13.8 mm³, *P*<0.05) but not in the SSCs-only group (152.0±21.9 mm³), as compared with the control group (145.8±10.1 mm³) (Fig. 1).

The results indicated that topically applied SSCs mixed with fibrin glue at infarct brain tissue after FCI injury significantly reduced the total infarcted volume by 40% and 42%, respectively, as compared to that of the control group and the SSCs-only group (Fig. 1).

**Example 2 Behavioural Tests**

Behavioral measurements were performed by the rotarod test and the grasping power test at the end of the 1st, 2nd, 3rd, and 4th weeks after SSCs administration on rats subjected to FCI injury.

*Rotarod Test*

For the rotarod test, an accelerating rotarod was used to assess motor deficit following ischemic injury in rats (R.J. Hamm et al., 1994, J. Neurotrauma 11: 187-196). The rats were placed on rungs of the accelerating rotarod and the amount of time the animals remained on the rotarod were measured. The speed was increased slowly from 4 rev/min to 40 rev/min over the course of 5 minutes. The time, in seconds, at which each animal fell off the rungs was recorded. Each animal received three consecutive trials.

Fig. 2 shows the results of the rotarod test of the four groups. The duration that the animals stayed on the rotarod was not significantly different among the four groups before surgical preparation. Sham-operated animals without ischemia stayed significantly longer on the rotarod test than other groups after FCI injury. After FCI injury, the mean latencies for rats to stay on the rotarod were 52%, 42% and 74% (p < 0.05 vs. control group and SSCs-only group) of baseline, respectively, in the control, SSCs-only and SSCs-fibrin glue groups at the end of the 1st week after SSCs administration, but 52%, 49% and 89% (p < 0.05 vs. control group and GDNF-only group) of baseline at the end of the 4th week after SSCs applied. The results showed that after FCI injury, the duration that rats stayed on the rotarod was significantly shorter in the control and SSCs-only groups than in that of SSCs-fibrin glue animals. These results indicated that cerebral injury area covered with fibrin glue containing SSCs could improve the balance and coordination of rats after FCI injury.

*Grasping Power Test*

The grasping power test was a modification of the method of Bertelli and Mira (J.A. Bertelli and J.C. Mira, 1995, J. Neurosci. Methods 58: 151-155). For the assessment of grasping strength, a bar of wires was connected to an ordinary electronic balance. Both forepaws were tested, one forepaw at a time. The untested forepaw was temporarily prevented from grasping by wrapping it with adhesive tape, and the tested forepaw was kept free. The rats were allowed to grasp the bar while being lifted by the tail with increasing firmness until they loosened their grip and the grasping power was scored.

The effects on grasping power test of the four groups are shown in Fig. 3. Among the four groups, there is no significant difference in the mean grasping power of the left forepaws of rats before the right MCA occlusion, and in that of the right forepaws of rats before or after right MCA occlusion. The mean value of grasping powers were 59%, 53% and 75% of baseline, respectively, in the control, SSCs-only and SSCs-fibrin glue groups at the end of the 1st week after SSCs administration, however, 58%, 63% and 94% (p<0.05 vs. control group) of baseline at the end of 4th week after SSCs application. These results showed that topical application of SSCs-fibrin glue is associated with improvement of grasping strength of rats after FCI injury.

**Example 3 Reverse Transcription-Polymerase Chain Reaction (RT-PCR) Analysis**

To confirm the neuronal regeneration and SSCs differentiations in the brain at 5 weeks after FCI injury, RT-PCR analysis using human glycerol-3-phosphate dehydrogenase (HG3PDH) was performed.

Total RNA was prepared from the brain of half of the animals from each group using the RNeasy purification system as described by the manufacturer (Qiagen; Valencia, CA; http://www.qiagen.com). Total RNA (1 µg) was reversely transcribed with Moloney murine leukemia virus reverse transcriptase at 42°C in the presence of oligo-dT primer. PCR was performed for the HG3PDH gene using specific primers designed from the published sequence of cDNA as follows: sense: 5'-GGCTGGGACTCATGGAGAT-3' (SEQ ID NO:1), and antisense: 5'-CGGGTAAGTCGTTGAGAAAG-3' (SEQ ID NO:2). Nested PCR was performed with the following primers: sense: 5'-TCTTGGAGAGCTGTGGTGTTG-3' (SEQ ID NO:3), and antisense: 5'- GTTACCTGAAAGGACTGC-3' (SEQ ID NO:4). The thermal profile was 1 minute at 95°C, 1 minute at 60°C, and 2 minutes at 72°C. The amplification cycles were all 35. To exclude possible contamination of genomic DNA, PCR was also applied to reactions without reverse transcription. The amplified complementary DNA was subjected to electrophoresis on a 3.5% polyacrylamide gel and visualized by silver staining.

HG3PDH was found in the brain of animals topically applied with SSCs (10⁶ cells) in fibrin glue (SSCs-fibrin glue group) one week after FCI injury and five weeks of reperfusion. However, no HG3PDH was detected in the control and the SSCs-only groups (Fig. 4).

As shown in the above examples, while topical application of the fibrin glue composition containing SSCs to ischemic brain significantly decreases the infarction volume in MCA occluded rats and also significantly improves the motor performance at the end of the 4th week after chronic FCI injury, the control group did not exhibit a neuroprotective effect in rats after chronic focal cerebral ischemia. The data indicate that the fibrin glue composition of the present invention is effective in treating the cerebral ischemia from MCA occlusion and enhancing recovery of motor function.

**Example 4 Fibrin Glue Composition Comprising SSCs and GDNF**

The following experiments were performed to investigate the therapeutic effects of a fibrin glue composition comprising not only SSCs, but also GDNF.

*Infarct Volume Analysis*

As described in Example 1, reproducible brain infarcts were obtained from a territory of right MCA occlusion in a GDNF-fibrin glue group, a SSCs-fibrin glue group, a GDNF-SSCs-fibrin glue group, and the control group. For the GDNF-fibrin glue group and GDNF-SSCs-fibrin glue group, 1 mg GDNF (RBI, Natick®, MA, USA) was mixed with the solution containing fibrinogen and aprotinin, with and without SSCs, respectively. The glues from the various groups were applied topically to the nerves as described above in Example 1.

Fig. 5 shows the infarct volumes assessed after one hour of MCA occlusion and five weeks of reperfusion by vital TTC staining. The infarct volumes of the GDNF-fibrin glue group, the SSCs-fibrin glue group, and the GDNF-SSCs-fibrin glue group were significantly reduced as compared with the control group. Each experimental group consisted of 6 rats. Results are expressed as mean ± SEM. *, *p*<0.05 when compared with the control group.

*Rotarod Test*

The rotarod test was performed as described in Example 2. Fig. 6 shows the results of the rotarod test of the five groups. The results are expressed as durations (in seconds) spent on the rotarod test. The duration that the animals stayed on the rotarod was not significantly different among the five groups before surgical preparation. Sham-operated animals without ischemia stayed significantly longer on the rotarod test than other groups after FCI injury. "0-week" indicates the time of topically applying the GDNF, SSCs or GDNF + SSCs. In the GDNF-fibrin glue group, the SSCs-fibrin glue group, and the GDNF-SSCs-fibrin glue group, the duration that rats stayed on the rotarod was significantly increased as compared with the control group at the end of the 1 st, 2nd, 3rd and 4th week after GDNF, SSCs or GDNF + SSCs administration. Each experimental group consisted of 6 rats. Results are expressed as mean ± SEM. *, *p*<0.05 when compared with the control group.

*Grapsing Power Test*

The grasping power test was also performed as described in Example 2. The effects on grasping power test of (A) right forepaw and (B) left forepaw on rats after FCI injury of the five groups are shown in Fig. 7. "0-week" indicates the time of topically applying the GDNF, SSCs or GDNF + SSCs. Among the five groups, there is no significant difference in the mean grasping power of the left forepaws of rats before the right MCA occlusion, or in the right forepaws of rats before or after right MCA occlusion. In the GDNF-fibrin glue group, the SSCs-fibrin glue group, and the GDNF-SSCs-fibrin glue group, the grasping power of left forepaw of rats was significantly increased as compared with the control group at the end of the 2nd, 3rd and 4th week after GDNF, SSCs or GDNF + SSCs administration. Each experimental group consisted of 6 rats. Results are expressed as mean ± SEM. *, *p*<0.05 when compared with the control group.

The above results showed that topical application of GDNF, SSCs or GDNF-SSCs-fibrin glue is associated with improvement of grasping strength of rats after FCI injury.

*Whishaw Reaching Test*

To further investigate the ability of the fibrin glue composition comprising SSCs + GDNF to enhance the functional recovery of nerves damaged by FCI injury, the whishaw reaching test was performed.

The whishaw reaching test is a modification of the method of Kolb and Cioe (B. Kolb and J. Cioe, 2000, Neuropharmacology 39(5): 756-64). Rats were food deprived to 85% body weight for the training and testing. Each animal was placed in the test cages (10×18×10 cm high) with floors and fronts constructed of 2 mm bars, 9 mm apart edge to edge. A 4-cm wide and 5-cm deep tray containing 45 mg food pellets was mounted in the front of each box. The rats are required to extend a forelimb through the gap in the bars, grasp and retract the food. The tray was mounted on runners and was retracted 0.5 cm from the cage so that the rats cannot scrape the food into the cage. If the animal attempts to rake the pellet out of the tray, the pellets will fall through the gap. An attempt is scored only when the rat reaches into the tray and touches the food pellets. If the rat reaches into the tray without touching a pellet, no attempt is scored.

The effects on the whishaw reaching test on rats after FCI injury of the five groups are shown in Fig. 8. "0-week" indicates the time of topically applying the GDNF, SSCs or GDNF + SSCs. In the GDNF-fibrin glue group and the SSCs-fibrin glue group, there is no significant difference in the test of rats before or after the right MCA occlusion. In the GDNF-SSCs-fibrin glue group, the scores of rats on the test were significantly increased as compared with the control group at the end of the 1 st, 2nd, 3rd and 4th week after GDNF + SSCs administration. Each experimental group consisted of 6 rats. Results are expressed as mean ± SEM. *, p<0.05 when compared with the control group.

It will be appreciated by those skilled in the art that changes could be made to the embodiments described above without departing from the broad inventive concept thereof. It is understood, therefore, that this invention is not limited to the particular embodiments disclosed, but it is intended to cover modifications of the present invention within, and as defined by, the appended claims.

## Claims

1. A fibrin glue composition or a topically applicable composition, suitable for repairing nerve damage and/or enhancing functional recovery of a damaged nerve, the composition comprising bone marrow stem cells (BMSCs).

2. A topical composition according to claim 1 comprising fibrin glue and/or an amount of BMSCs effective to at least one of repair the nerve damage and enhance at least partially the functional recovery of the nerve.

3. A composition according any preceding claim, wherein the amount of BMSCs comprises from 10⁵ to 10⁷ cells.

4. A composition according to claim 5, wherein the amount of BMSCs comprises about or from 10⁶ cells.

5. A composition according any preceeding claim, wherein the BMSCs are size-sieved stem cells (SSCs).

6. A composition according to claim 3, wherein the SSCs are sieved with a 3 µm porous sieve.

7. A composition according any preceding claim, further comprising an amount of a nerve growth factor, preferably effective to repair nerve damage and/or enhance the functional recovery of the nerve.

8. A composition according any preceding claim additionally comprising a nerve growth factor that is glial cell line-derived neurotrophic factor (GDNF) or pituitary adenylate cylase-activating polypeptide (PACAP).

9. A composition according to claim 7 or 8, wherein the nerve growth factor is present at a concentration of from 1 µg/ml to 1000 µg/ml of the composition, optionally at about 50 µg/ml of the composition.

10. The use of a composition according to any of claims 1 to 9, in the manufacture of a medicament for repairing nerve damage and/or enhancing functional recovery of a damaged nerve.

11. The use according to claim 10, wherein the nerve damage is caused by focal cerebral ischemia or is a result of an ischemic brain disease.

12. The use according to claim 11, wherein the focal cerebral ischemia is chronic focal cerebral ischemia and/or the ischemic brain disease is stroke, thrombosis and/or embolization of the middle cerebral artery.

13. The use according to claim 10, wherein the nerve is a central nervous system nerve.

14. The use according to any of claims 10 to 13, wherein the composition is prepared fresh right or just before use.

15. A composition according to any of claims 1 to 9 for use in a method of treatment (e.g. by therapy) of the human or animal body.

16. A composition according to any of claims 1 to 9 for repairing nerve damage and/or enhancing functional recovery of a damaged nerve.


SEQUENCE LISTING

<110>  Cheng, Henrich

<120>  Composition for Repairing Nerve Damage

<130>  N99808

<150>  USA 11/516,378
<151>  2006-09-06

<160>  4

<170>  PatentIn version 3.3

<210>  1
<211>  19
<212>  DNA
<213>  Artificial

<220>
<223>  PCR primer

<400>  1
ggctgggact catggagat                                                    19

<210>  2
<211>  20
<212>  DNA
<213>  Artificial

<220>
<223>  PCR primer

<400>  2
cgggtaagtc gttgagaaag                                                   20

<210>  3

<211>  21

<212>  DNA

<213>  Artificial

<220>

<223>  PCR primer

<400>  3

tcttggagag ctgtggtgtt g                                                21

<210>  4

<211>  18

<212>  DNA

<213>  Artificial

<220>

<223>  PCR primer

<400>  4

gttacctgaa aggactgc                                                    18